(19) Europäisches Patentamt
European Patent Office
Office européen des brevets

(11) Veröffentlichungsnummer : **0 101 990**
**B1**

(12) # EUROPÄISCHE PATENTSCHRIFT

(45) Veröffentlichungstag der Patentschrift : **11.12.85**

(51) Int. Cl.⁴ : **C 07 C127/22**, A 01 N 47/34

(21) Anmeldenummer : **83107789.6**

(22) Anmeldetag : **08.08.83**

(54) **N-Benzoyl-N'-phenoxyphenylharnstoffe, Verfahren zu ihrer Herstellung und ihre Verwendung zur Bekämpfung von Schädlingen.**

(30) Priorität : **31.08.82 DE 3232265**

(43) Veröffentlichungstag der Anmeldung :
**07.03.84 Patentblatt 84/10**

(45) Bekanntmachung des Hinweises auf die Patenterteilung : **11.12.85 Patentblatt 85/50**

(84) Benannte Vertragsstaaten :
**AT BE CH DE FR GB IT LI NL SE**

(56) Entgegenhaltungen :
**EP-A- 0 057 888**
**FR-A- 2 300 076**
**FR-A- 2 318 153**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Lange, Arno, Dr.**
**Oberes Geistal 3b**
**D-6702 Bad Duerkheim (DE)**
Erfinder : **Adolphi, Heinrich, Dr.**
**Kalmitweg 11**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 101 990 B1

**Beschreibung**

Es ist bekannt, daß N-Benzoyl-N'-phenyl- bzw. -phenoxyphenylharnstoffe als Insektizide verwendet werden können (J. Agr. Food Chem. *21,* 348 (1973) ; DE-OS 2 531 202).

Aus der EP-A- 0 057 888 sind insbesondere N-Benzoyl-N'-phenoxyphenylharnstoffe bekannt, die der Formel

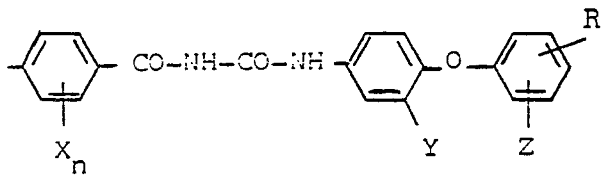

entsprechen, wobei speziell Z Chlor in 3-Stellung oder Methyl bedeutet ; diese gegenüber den ursprünglich bekanntgewordenen Wirkstoffen bereits fortentwickelten Wirkstoffe erscheinen jedoch weiter verbesserungsbedürftig.

Dabei hat sich gezeigt, daß durch gezielte Einführung eines bestimmten weiteren Substituenten in den Phenylteil eine größere Variationsbreite des Substitutionsmusters im Phenoxyteil erreicht wird und gegebenenfalls auch auf eine solche Substitution im Phenoxyteil ganz oder teilweise verzichtet werden kann, was die Herstellung der Wirkstoffe erleichtert.

Es wurde gefunden, daß N-Benzoyl-N'-phenoxyphenylharnstoffe der Formel (I)

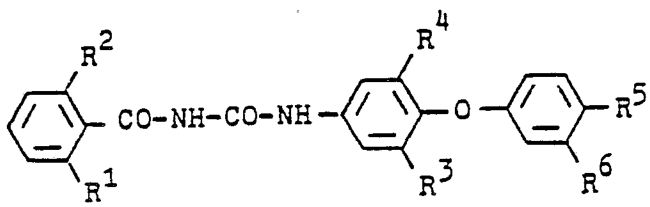

(I)

in der

$R^1$ Fluor, Chlor oder Methyl ;
$R^2$ Fluor, Chlor oder Wasserstoff ;
$R^3$ Chlor oder Brom ;
$R^4$ Chlor oder Brom und
$R^5$ und $R^6$ Fluor, Chlor, Brom, Trifluormethyl, Wasserstoff bedeuten, wobei
$R^5$ und $R^6$ gleich oder verschieden sein können,

Schädlinge, insbesondere Insekten, wirksamer bekämpfen als bekannte N-Benzoyl-N'-phenoxyphenylharnstoffe.

Bevorzugt sind solche Verbindungen, bei denen mindestens ein Substituent $R^5$ bzw. $R^6$ Wasserstoff ist, d. h. der Phenoxyrest ist vorzugsweise unsubstituiert oder in 3- oder 4-Stellung monosubstituiert.

Man erhält solche N-Benzoyl-N'-phenoxyphenylharnstoffe durch Umsetzen entweder eines entsprechenden Phenoxyanilins (II)

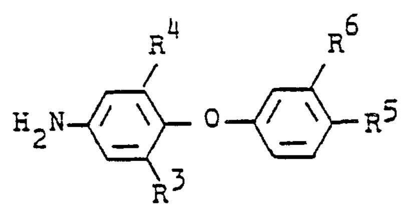

(II)

mit einem entsprechenden Benzoylisocyanat (III)

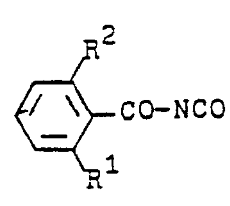

(III)

oder eines Phenoxyphenylisocyanats (IV)

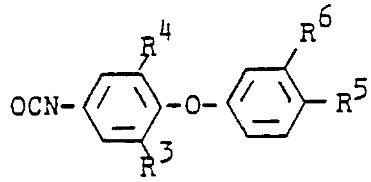

(IV)

mit einem Benzamid (V)

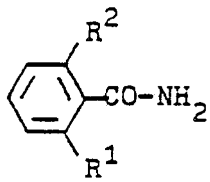

(V)

Die Ausgangsstoffe sind entweder bekannt oder können mit fachüblichen Methoden leicht hergestellt werden.

Die Herstellung der Benzoylisocyanate (II) kann z. B. nach den Vorschriften in J. Org. Chem. *28*, 1805-1811 (1963) oder J. Agr. Food Chem. *21*, 348 (1973) geschehen.

Für die Herstellung der Phenylisocyanate sind z. B. die Angaben bei Weygand-Hilgetag, Organisch-Chemische Experimentierkunst, 1970 oder in der DE-OS 2 538 178 nützlich. Für die Herstellung der Diphenylether kann man auf die Angaben z. B. bei Barry et al. Pr. Irish Acad. 53 B, 61 66, 82 (1950) zurückgreifen.

Die vorstehend angegebenen Umsetzungen laufen unter den in der DE-OS 2 531 202 beschriebenen Bedingungen ab und bieten keine besonderen Schwierigkeiten.

Die erfindungsgemäßen Verbindungen fallen in der Regel analysenrein an ; andernfalls können sie durch Umkristallisieren gereinigt werden. Zu ihrer Charakterisierung dienen Elementaranalyse, Schmelzpunkt, IR- und NMR-Spektrum.

Zur Durchführung der erstgenannten Verfahrensvariante wird zweckmäßig das substituierte Anilin zusammen mit dem Lösungs- oder Verdünnungsmittel vorgelegt, dann wird eine stöchiometrische Menge an Isocyanat zugegeben. Nach in der Regel nicht mehr als zwei Stunden hat sich das Umsetzungsprodukt entweder als kristalliner Niederschlag abgesetzt, der abgesaugt wird oder er bleibt in Lösung. In diesem Fall wird unter vermindertem Druck eingeengt und getrocknet. Bei der anderen Verfahrensvariante beträgt die Reaktionsdauer im allgemeinen über 2 bis etwa 6 Stunden. Die vorstehenden Zeitangaben beziehen sich auf Raumtemperatur. Ein katalysator wie Triethylamin oder Dibutylzinndiacetat kann vorteilhaft sein.

Herstellbeispiel

a) 4,3 g 3,5-Dichlor-4-phenoxyanilin wird in 50 ml Toluol vorgelegt. Man setzt bei Raumtemperatur allmählich 3,3 g 2,6-Difluorbenzoylisocyanat zu, wobei sich die Temperatur von 22° auf 32° erhöht. Man rührt über Nacht nach, saugt ab und trocknet i.V. 6,7 g N-2,6-Di-fluorbenzoyl-N'-(3,5-dichlor-4-phenoxy)-phenylharnstoff vom Schmelzpunkt Fp. : 195-198 °C.

b) 4,3 g 3,5-Dichlor-4-(4-chlorphenoxy)-anilin werden in 50 ml Toluol vorgelegt. Man setzt bei Raumtemperatur 2,9 g 2-Chlorbenzoylisocyanat in 5 ml Toluol zu, wobei sich die Temperatur von 23° auf 31° C erhöht. Man rührt über Nacht nach, saugt ab und trocknet i.V. 6,2 g N-2-Chlorbenzoyl-N'-[3,5-dichlor-4-(4-chlorphenoxy)]-phenylharnstoff vom Schmelzpunkt Fp. : 208-211 °C.

Entsprechend wurden die folgenden Verbindungen erhalten ; dabei entspricht Herstellbeispiel a) der Nr. 5 und Herstellbeispiel b) der Nr. 4 der folgenden Tabelle.

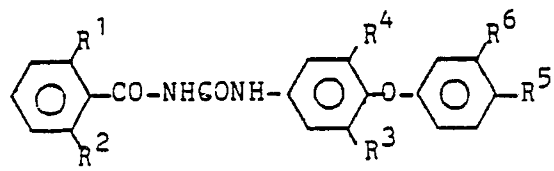

| Nr. | $R_1$ | $R_2$ | $R_3$ | $R_4$ | $R_5$ | $R_6$ | Fp |
|---|---|---|---|---|---|---|---|
| 1 | F | F | Cl | Cl | Cl | H | 199 - 202 |
| 2 | Cl | Cl | Cl | Cl | Cl | H | 229 - 232 |

(Fortsetzung)

| Nr. | $R_1$ | $R_2$ | $R_3$ | $R_4$ | $R_5$ | $R_6$ | Fp |
|---|---|---|---|---|---|---|---|
| 3 | Cl | F | Cl | Cl | Cl | H | |
| 4 | Cl | H | Cl | Cl | Cl | H | 208 - 211 |
| 5 | F | F | Cl | Cl | H | H | 195 - 198 |
| 6 | Cl | Cl | Cl | Cl | H | H | 216 - 219 |
| 7 | Cl | H | Cl | Cl | H | H | 208 -212 |
| 8 | F | F | Cl | Cl | F | H | 201-203 |
| 9 | Cl | Cl | Cl | Cl | F | H | 211-214 |
| 10 | Cl | H | Cl | Cl | F | H | 174-178 |
| 11 | F | F | Cl | Cl | Br | H | 210-215 |
| 12 | Cl | Cl | Cl | Cl | Br | H | 230-234 |
| 13 | Cl | H | Cl | Cl | Br | H | 217-220 |
| 14 | F | F | Cl | Cl | $CF_3$ | H | 192-195 |
| 15 | Cl | Cl | Cl | Cl | $CF_3$ | H | 180-185 |
| 16 | Cl | H | Cl | Cl | $CF_3$ | H | 212-218 |
| 17 | F | F | Cl | Cl | H | Cl | 192-194 |
| 18 | Cl | Cl | Cl | Cl | H | Cl | 204-210 |
| 19 | Cl | H | Cl | Cl | H | Cl | 198-205 |
| 20 | F | F | Cl | Cl | H | $CF_3$ | 170-174 |
| 21 | Cl | Cl | Cl | Cl | H | $CF_3$ | 189-195 |
| 22 | Cl | H | Cl | Cl | H | $CF_3$ | 187-190 |
| 23 | F | F | Br | Cl | H | H | 175-178 |
| 24 | Cl | Cl | Br | Cl | H | H | 205-209 |
| 25 | Cl | H | Br | Cl | H | H | 205-210 |
| 26 | F | F | Br | Cl | Cl | H | 205-208 |
| 27 | Cl | Cl | Br | Cl | Cl | H | 245-249 |
| 28 | Cl | H | Br | Cl | Cl | H | 210-214 |
| 29 | F | F | Br | Br | H | H | 178-185 |
| 30 | Cl | Cl | Br | Br | H | H | 225-228 |
| 31 | Cl | H | Br | Br | H | H | 220-224 |
| 32 | F | F | Br | Br | Cl | H | 206-210 |
| 33 | Cl | Cl | Br | Br | Cl | H | 244-248 |
| 34 | Cl | H | Br | Br | Cl | H | 212-218 |

Die N-Benzoyl-N'-phenoxyphenylharnstoffe der Formel I sind geeignet, Schädlinge vor allem aus der Klasse der Insekten wirksam zu bekämpfen. Sie können im Pflanzenschutz sowie auf dem Hygiene-, Vorratsschutz- und Veterinärsektor als Schädlingsbekämpfungsmittel eingesetzt werden. Ein besonders wichtiges Anwendungsgebiet der erfindungsgemäßen Mittel sind Raupen von Schadschmetterlingen.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostriana nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarus (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling) ;

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varicestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner) ;

aus der Ordnung der Zweiflügler (Diptera) beispielsweise Lycoria pectoralis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus olea (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ocis, Chrysomya macellaria, Chrysomya hominivorax, Lucilia cuprina, Lucilia sericata, Hypoderma lineata ;

aus der Ordnung der Hautflügler (Hymenoptera) beispielsweise Athalia rosae (Rübenblattwespe), Hoplocampa minuta (Pflaumensägewespe), Monomorium pharaonis (Pharaomeise), Solenopsis geminata (Feuerameise), atta sexdens (Blattschneiderameise) ;

aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze) ;

aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Roside Apfellaus), Sappaphis mala (Mehlige Birnblattlaus), Dysphis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthoriphon

onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia picea (Weißtannenstammlaus), Adelges laricis (Rote Fichtengallenlaus), Viteus vitifolii (Reblaus) ;

aus der Ordnung der Termiten (Isoptera) beispielsweise Reticulitermes lucifugus, Calotermes flavicollis, Leucotermes flavipes, Termes natalensis ;

aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melano plus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Die Mittel können auch verwendet werden zur Bekämpfung z. B. von Spinnentieren (Arachnoidea bzw. Acarina) wie Ixodes ricinus (Holzbock), Ornithodorus moubata, Amblyomma americanum, Dermacentor silvarum, Boophilus microplus, Tetranychus telarius, Tetranychus atlanticus, Tetranychus pacificus, Paratetranychus pilosus, Bryobia praetiosa ; ferner zur Bekämpfung von Fadenwürmern (Nemathelminthes) wie Wurzelgallennematoden, z. B. Meloidogyne incognita, Meloidogyne hapla, Meloidogyne javanica, Zysten bilbende Nematoden, z. B. Heterodera rostochiensis, Heterodera schachtii, Heterodera avenae, Heterodera glycines, Heterodera triflolii, Stock- und Blattälchen, z. B. Ditylenchus dipsaci, Ditylenchus destructor, Paratylenchus neglectus, Paratylenchus penetrans, Paratylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfingungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldisperionen können die Substanzen als solche oder in einem Öl oder Lösungsmittel gelöst, mittels Netz-, Haft-, Dispergier- oder Emulgiermitel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind :

I. 5 Gewichtsteile der Verbindung Nr. 1 werden mit 95 Gewichtsteilen feinteiligem Kaolin innig vermischt. Man erhält auf diese Weise ein Stäubemittel, das 3 Gew.% des Wirkstoffs enthält.

II. 30 Gewichtsteile der Verbindung Nr. 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gewichtsteile der Verbindung Nr. 4 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung Nr. 13 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

V. 80 Gewichtsteile der Verbindung Nr. 19 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.% Wirkstoff, vorzugsweise zwischen 0,5 und 90 Gew.%.

Die Wirkstoffkonzentrationen in den anwendungsfertigen Zubereitungen können in größeren Bereichen variiert werden.

Im allgemeinen liegen sie zwischen 0,000 1 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,2 bis 10, vorzugsweise 0,5 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmitelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden : 1,2-Dibrom-3-chlorpropan, 1,3-Dichlorpropen, 1,3-Dichlorpropen + 1,2-Dichlorpropan, 1,2-Dibrom-ethan, 2-sec.-Butylphenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethylbenzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N-[(methylcarbamoyl)-oxyl]-thio-acetimidat, Methyl-N',N'-dimethyl-N-[(methylcarbamoyl)oxyl]-1-thiooxamidat, N-(2)Methyl-chlor-phenyl)-N',N'-dimethylformamidin, Tetrachlorthiophen, 1-(2,6-Difluor-benzoyl)-3-(4-chlor-phenyl)-harnstoff, O,O-Dimethyl-O-(p-nitrophenyl)-phosphorthioat, O,O-Diethyl-O-(p-nitrophenyl)-phosphorthioat, O-Ethyl-O-(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)-phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphonothioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-[p-methylsulfinyl-phenyl]-phosphorthioat, O-Ethyl-S-phenyl-ethyl-phosphonodithioat, O,O-Diethyl-[2-chlor-1-(2,4-dichlorphenyl)-vinyl]-phosphat, O,O-Dimethyl-[-2-chlor-1-(2,4,5-trichlorphenyl)]-vinyl-phosphat, O,O-Dimethyl-S-(1'-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O,O-Dimethyl-O-2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoyl-methyl)-phosphordithioat, O,O-Dimethyl-S-(N-methyl-carbamoyl-methyl)-phosphorthioat, O,O-Dimethyl-S-(N-methoxyethyl-carbamoyl-methyl)-phosphordithioat, O,O-Dimethyl-S-(N-formyl-N-methyl-carbamoylmethyl-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthio-methyl)-phosphordithioat, O,O-Diethyl-S-[(p-chlorphenylthio)-methyl]-phosphorthioat, O,O-Dimethyl-S-(2-ethylthio-ethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinyl-ethyl)-phosphorthioat, O,O-Diethyl-thiophosphoryliminophenyl-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophos-

phat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O-[2-isopropyl-4-methyl-pyrimidinyl(6)]-phosphorthioat, O,O-Diethyl-O-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amido-thioat, O,S-Dimethyl-N-acetyl-phosphoramidothioat, γ-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,-trans-chrysanthemat, 5-Benzyl-furyl-(3)-methyl-DL-cis,-trans-chrysanthemat, 3-Phenoxybenzyl(±)-cis, trans-2,2-dimethyl-3-(2,2-dichlorvinyl)-cyclopropancarboxylat, α-Cyano-3-phenoxybenzyl(±)-cis,trans-2,2-dimethyl-3-(2,2-dichlorvinyl)-cyclopropancarboxylat, (s)-α-Cyano-3-phenoxybenzyl-cis(1R,3R)-2,2-dimethyl-3-(2,2-dibromvinyl)-cyclopropancarboxylat, 3,4,5,6-Tetrahydrophthalimido-ethyl-DL-cis, trans-chrysanthemat, 2-Methyl-5-(2-propinyl)-3-furylmethyl-chrysanthemat, (α-Cyano-3-phenoxybenzyl)-α-isopropyl-4-chlorphenylacetat.

Wie sich aus den nachfolgenden Beispielen ergibt, haben die erfindungsgemäßen N-Benzoyl-N'-phenoxy-phenylharnstoffe eine bei vielen Arten deutlich bessere, bei den meisten Arten mindestens gleichwertige Wirkung gegenüber vergleichbaren bekannten Mitteln, wobei zum Vergleich in den nachstehenden Anwendungsbeispielen das Handelsprodukt Diflubenzuron der Formel

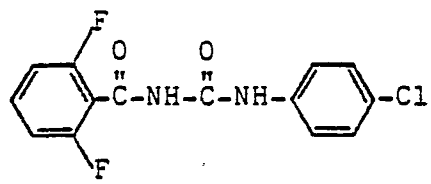

gewählt wurde.

Anwendungsbeispiel 1

Zuchtversuch mit Moskito-Larven (Aedes aegypti)

200 ml Leitungswasser werden mit der Wirkstoffaufbereitung versetzt und darauf mit 30 bis 40 Aedes-Larven im 4. Stadium belegt.

Die Versuchstemperatur beträgt 25 °C. Beurteilt wird Verpuppung und Schlüpfen der Imagines, wobei eine unbehandelte Kontrolle als Maßstab dient.

Während der Versuchsdauer wird einmal mit einem handelsüblichen Aufzuchtfutter für Aquarienfische gefüttert. Danach haben die Verbindungen Nr. 1, 2, 4, 5 und 8 bei einer Konzentration von 0,001 bis 0,04 ppm absolut tödliche Wirkung ; das Verbleichsmittel hat in diesem Falle unter vergleichbaren Bedingungen ähnliche Wirkung ; einzelne erfindungsgemäße Verbindungen sind deutlich überlegen.

Anwendungsbeispiel 2

Zuchtversuch mit Maiszünsler-Larven (Ostrinia nubilalis)

Die Zucht erfolgt auf einem Nährboden folgender Zusammensetzung :

515 g Maismehl
130 g Weizenkeime
137 g Bierhefe
18 g Ascorbinsäure
10 g Cellulosepulver
5 g Nipagin
20 g Wessons Salz
20 ml Vitaminlösung
80 g Agar
3 100 ml Wasser

Je 50 ml füllt man in 100 ml Plastikbecher und mischt die wäßrige Wirkstoffaufbereitung sorgfältig unter.

Nach Erkalten belegt man jedes Gefäß mit 4 Raupen (L 3). Pro Konzentration werden 5 Gefäße angesetzt.

Die Beobachtung erstreckt sich bis zum Schlüpfen der Falter.

In diesem Versuch haben die Verbindungen Nr. 1, 2 und 4 bei einer Konzentration zwischen 0,4 und 4,0 ppm 100 % Mortalität zur Folge, das Mittel Nr. 5 erzielt bei 1,0 ppm noch 92 % Mortalität, während das Vergleichsmittel noch bei 5,0 ppm keine absolut tödliche Wirkung hat.

Anwendungsbeispiel 3

Zuchtversuch mit Baumwollwanzen (Dysdercus intermedius)

Baumwollwanzen Dysdercus intermedius werden im 4. Larvenstadium in Petrischalen (∅ 10 cm) dem Wirkstoffbelag der Testsubstanz für 24 Stunden ausgesetzt.

Die Überlebenden züchtet man in 1-1-Gläsern auf feuchtem Quarzsand, der vorher mit der Wirkstofflösung versetzt wurde bis zum Schlüpfen der $F_1$-Generation.

Dabei entsprechen in der Behandlung

| | |
|---|---|
| 2,5 mg/Schale | 25 ppm im Sand |
| 1,0 mg/Schale | 10 ppm im Sand |
| 0,5 mg/Schale | 5 ppm in Sand |
| usw. | |

Beurteilt wird Mortalität und Vermehrung.

In diesem Versuch erweisen sich die beanspruchten Verbindungen Nr. 1 und 4 als zwei- bis fünfmal wirksamer als das Vergleichsmittel.

Anwendungsbeispiel 4

Zuchtversuch mit Stubenfliegen-Larven (Musca domestica)

je 4,5 ml Magermilch füllt man in 50 ml Penicillingläser und versetzt sie darauf mit 0,5 ml der wäßrigen Wirkstoffaufbereitung. Nach kurzem Mischen fügt man ein Wattebällchen dazu und belegt dieses mit ca. 50 Ei-Larven der Stubenfliege.

Die Gläser lagert man abgedeckt bei Raumtemperatur und beurteilt die Entwicklung nach 7 Tagen.

In diesem Versuch erzielen die Verbindungen Nr. 1, 4 und 5 bei einer Anwendungskonzentration zwischen 5 und 20 ppm absolut tödliche Wirkung.

**Patentansprüche**

1. N-Benzoyl-N'-phenoxyphenylharnstoffe der Formel (I)

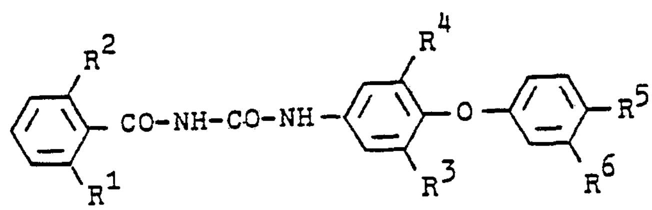

(I)

in der

$R^1$ Fluor, Chlor oder Methyl ;

$R^2$ Fluor, Chlor oder Wasserstoff ;

$R^3$ Chlor oder Brom ;

$R^4$ Chlor oder Brom und

$R^5$ und $R^6$ Fluor, Chlor, Brom, Trifluormethyl, Wasserstoff

bedeuten, wobei

$R^5$ und $R^6$ gleich oder verschieden sein können.

2. Verfahren zur Herstellung von N-Benzoyl-N'-phenoxyphenylharnstoffen der Formel I gemäß Anspruch 1, dadurch gekennzeichnet, daß man entweder ein entsprechend substituiertes Phenoxyanilin (II).

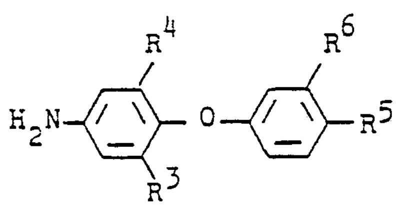

(II)

mit einem entsprechenden Benzoylisocyanat (III)

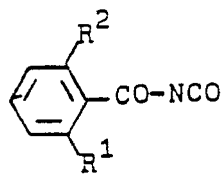

(III)

oder ein Phenoxyphenylisocyanat (IV),

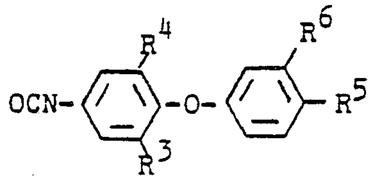

(IV)

mit einem Benzamid (V)

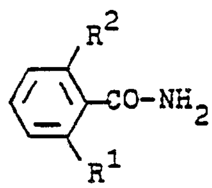

(V)

umsetzt.

3. Mittel zur Bekämpfung von Schädlingen, enthaltend einen N-Benzoyl-N'-phenoxyphenylharnstoff der Formel I gemäß Anspruch 1.

4. Mittel zur Bekämpfung von Schädlingen, enthaltend einen festen oder flüssigen Trägerstoff und/oder einen Synergisten und einen N-Benzoyl-N'-phenoxyphenylharnstoff der Formel I gemäß Anspruch 1.

5. Verwendung von N-Benzoyl-N'-phenoxyphenylharnstoffen der Formel I gemäß Anspruch 1 zur Bekämpfung von Schädlingen.

6. Verfahren zur Bekämpfung von Schädlingen, dadurch gekennzeichnet, daß man eine wirksame Menge eines N-Benzoyl-N'-phenoxyphenylharnstoffs der Formel I gemäß Anspruch 1 auf die Schädlinge und/oder deren Lebensraum einwirken läßt.

**Claims**

1. An N-benzoyl-N'-phenoxyphenylurea of the formula (I)

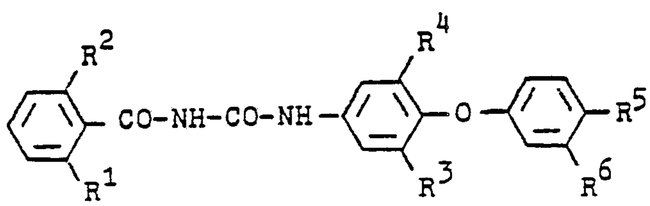

(I)

where

$R^1$ is fluorine, chlorine or methyl,

$R^2$ is fluorine, chlorine or hydrogen,

$R^3$ and $R^4$ are each chlorine or bromine, and

$R^5$ and $R^6$ can be identical or different and are each fluorine, chlorine, bromine, trifluoromethyl or hydrogen.

2. A process for the manufacture of N-benzoyl-N'-phenoxy-phenylureas of the formula I as claimed in claim 1, wherein either an appropriately substituted phenoxyaniline (II)

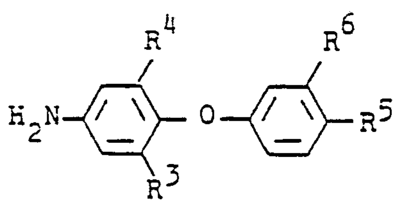

(II)

is reacted with an appropriate benzoyl isocyanate (III)

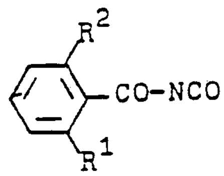

(III)

or a phenoxyphenyl isocyanate (IV)

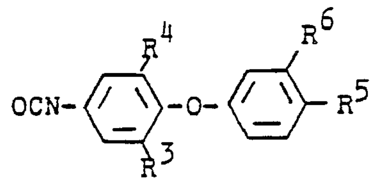

(IV)

is reacted with a benzamide (V)

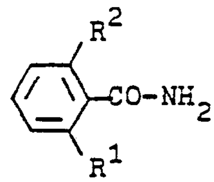

(V)

3. An agent for combating pests, containing an N-benzoyl-N'-phenoxyphenylurea of the formula I as claimed in claim 1.

4. An agent for combating pests, containing a solid or liquid carrier and/or a synergist, and an N-benzoyl-N'-phenoxyphenylurea of the formula I as claimed in claim 1.

5. The use of an N-benzoyl-N'-phenoxyphenylurea of the formula I as claimed in claim 1 for combating pests.

6. A process for combating pests, wherein an effective amount of an N-benzoyl-N'-phenoxyphenylurea of the formula I as claimed in claim 1 is allowed to act on the pests and/or their habitat.

**Revendications**

1. N-benzoyl-N'-phénoxyphénylurée de formule (I)

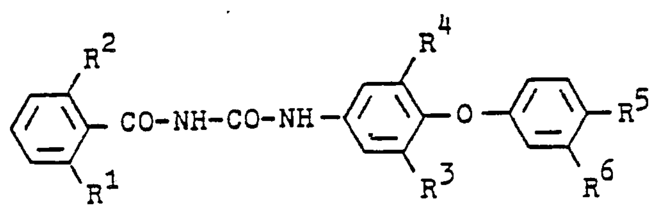

(I)

dans laquelle

$R^1$ représente fluor, chlore ou méthyle,
$R^2$ représente fluor, chlore ou hydrogène,
$R^3$ représente chlore ou brome,
$R^4$ représente chlore ou brome et
$R^5$ et $R^6$ représentent fluor, chlore, brome, trifluorométhyle, hydrogène
$R^5$ et $R^6$ pouvant être identiques ou différents.

2. Procédé de préparation de N-benzoyl-N'-phénoxyphénylurée de formule (I) selon la revendication 1, caractérisé par le fait qu'on fait réagir, ou bien une phénoxyaniline substituée de façon correspondante (II).

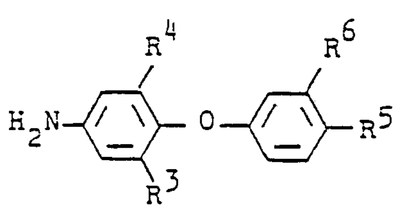

(II)

avec un isocyanate de benzoyle correspondant (III)

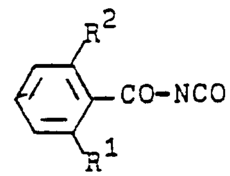

(III)

ou un isocyanate de phénoxyphényle (IV)

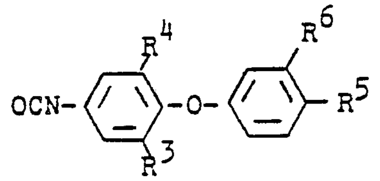

(IV)

avec un benzamide (V)

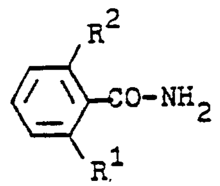

(V)

3. Moyen de lutte contre les insectes nuisibles contenant une N-benzoyl-N'-phénoxyphénylurée de formule I selon la revendication 1.

4. Moyen de lutte contre les insectes nuisibles contenant un support solide ou liquide et/ou une N-benzoyl-N'-phénoxyphénylurée de formule I selon la revendication 1.

5. Utilisation de N-benzoyl-N'-phénoxyphénylurées de formule I selon la revendication 1 pour la lutte contre les insectes nuisibles.

6. Procédé de lutte contre les insectes nuisibles, caractérisé par le fait qu'on fait agir une quantité efficace d'une N-benzoyl-N'-phénoxyphénylurée de formule I selon la revendication 1 sur les insectes nuisibles et/ou leur biotope.